(19) Europäisches Patentamt
European Patent Office
Office européen des brevets

(11) Publication number: 0 132 544 B1

(12) # EUROPEAN PATENT SPECIFICATION

(45) Date of publication of patent specification: 04.11.87

(51) Int. Cl.⁴: C 07 C 17/08

(21) Application number: 84106224.3

(22) Date of filing: 30.05.84

(54) ## Hydrohalogenation of conjugated dienes.

(30) Priority: 20.07.83 US 515562

(43) Date of publication of application: 13.02.85 Bulletin 85/07

(45) Publication of the grant of the patent: 04.11.87 Bulletin 87/45

(84) Designated Contracting States: BE CH DE FR GB LI NL

(56) References cited:
DE-B-1 253 264
US-A-3 812 212

(73) Proprietor: Union Camp Corporation
1600 Valley Road
Wayne New Jersey 07470 (US)

(72) Inventor: Mitchell, Peter W.D.
106 Lancaster Road
Freehold, NJ 07728 (US)
Inventor: McElligott, Lois T.
1158 Wheatsheaf Lane
Abington, PA 19001 (US)
Inventor: Sasser, David E.
6290 Nancy Drive
Jacksonville, FA 32210 (US)

(74) Representative: von Füner, Alexander, Dr. et al
Patentanwälte v. Füner, Ebbinghaus, Finck
Mariahilfplatz 2 & 3
D-8000 München 90 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 132 544 B1

Courier Press, Leamington Spa, England.

**Description**

## BACKGROUND OF THE INVENTION

Field of the Invention

The invention relates to processes for the hydrohalogenation of conjugated diene hydrocarbons.

Brief Description of the Prior Art

The literature is replete with descriptions of processes for the hydrohalogenation of conjugated dienes. Representative of such descriptions are those found in the U.S. Patents 2,882,323 and 3,016,408 and in British Patent 896,262.

The U.S. Patent 3,812,212 describes the free radical addition of hydrogen bromide to monoolefins in the presence of a quaternary salt. This mechanism of reaction would be undesirable in the present process of the invention because the free radical addition of hydrogen chloride to myrcene (a conjugated diene) gives products other than the products desired in the present process of the invention. The myrcene would extensive polymerize under the reaction conditions suggested by this patent reference.

Auslegeschrift 1,253,264 describes the gas phase reaction of butadiene with hdyrogen chloride over a supported cuprous chloride catalyst. The temperatures employed are within the range of from 50 to 150°C. The temperature required to process myrcene in the vapor phase would be extremely high, i.e., 167°C. At this temperature and in the presence of acid, no useful product would be obtained.

The present invention is particularly advantageous when used to hydrochlorinate myrcene. Myrcene is a conjugated diene of the formula:—

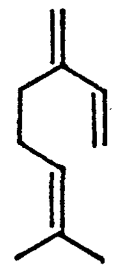

(I)

When hydrochlorinated in the absence of any catalyst, the major product is myrcenyl chloride. However, the commercially valuable products of myrcene hydrochlorination are the associated co-products, namely, geranyl chloride and neryl chloride. Hydrochlorination in the presence of a copper catalyst shifts the reaction in favor of the desired co-products. It has been postulated that the hydrochlorination of myrcene in the presence of a copper catalyst proceeds according to the reaction scheme:—

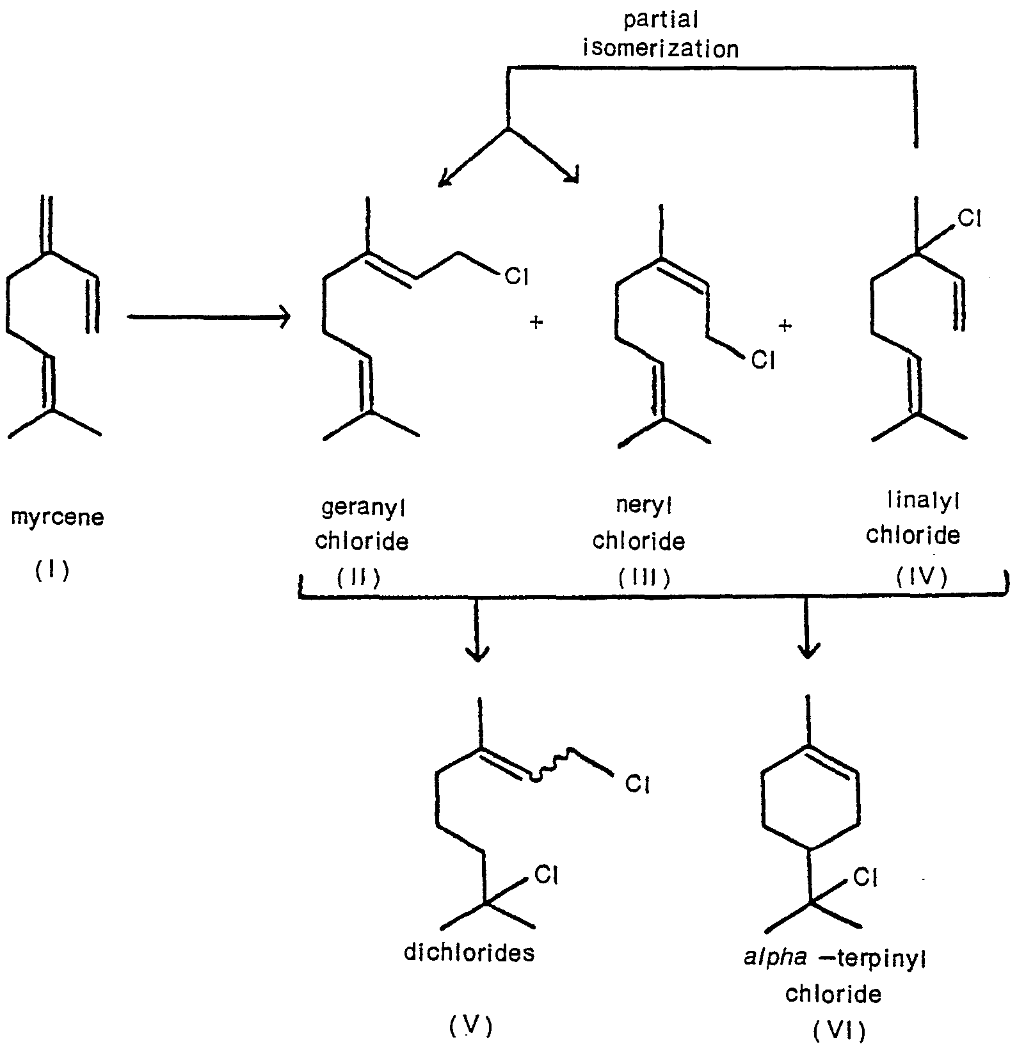


myrcene (I) geranyl chloride (II) + neryl chloride (III) + linalyl chloride (IV)

dichlorides (V) *alpha*–terpinyl chloride (VI)

Cyclization may also undesirably occur to form the *alpha*-terpinyl chlorides.

When the copper catalyst employed is in the form of cupric chloride ($CuCl_2$) the products generally include substantial proportions of linalyl chloride and lesser proportions of the desired geranyl and neryl chlorides. When the copper catalyst is in the form of cuprous chloride, the linalyl chloride product is lessened due, apparently, to partial isomerization to the desired geranyl and neryl chlorides.

From the above proposed reaction scheme, it will be appreciated that any process for hydrochlorination of myrcene, to be commercially feasible, must result in a favorable yield of the desired geranyl (II) and neryl (III) monochlorides and minimal formation of linalyl (IV) and *alpha*-terpinyl (VI) monochlorides. It was previously appreciated that the relative proportions of monochlorides (II), (III) and (IV) in the hydrochlorination product reaction mixture could be controlled to some degree by selection of the reaction temperature, gas flowrate and catalyst concentration.

We have now found that when the prior art hydrohalogenation of a conjugated diene, such as the hydrochlorination of isoprene and in particular the hydrochlorination of myrcene, is carried out in the presence of an organic quaternary salt, then the isomerization of the allylic chloride products during the hydrohalogenation reaction is shifted to favor formation of the less-substituted allylic chloride, being geranyl chloride (II) and neryl chloride (III) in the above example.

The advantages associated with the improved process of the invention are improved overall yield of the more desirable chloride, for example prenyl chloride from isoprene and neryl and geranyl chlorides from myrcene, and for the latter case, a greater selectivity of the more important geranyl isomer.

## SUMMARY OF THE INVENTION

The invention comprises, in a method for the hydrohalogenation of a conjugated diene which comprises, hydrohalogenating the diene in the presence of a catalytic proportion of a hydrohalogenation catalyst, the improvement which comprises; carrying out the hydrohalogenation in the presence of an organic quaternary salt.

The improved process of the invention may be carried out in a batch or a continuous manner.

In an improved embodiment the organic quaternary salt has a carbon atom content of at least 18 whereby especially preferred are trioctylmethylammonium chloride, a mixture of tri(alkyl)methylammonium chlorides where the alkyl portion comprises of a chain of from eight to ten carbon atoms, benzyldimethylstearylammonium chloride, benzylhydroxyethyl(2)coco imidazolium chloride, tetra(octadecyl)ammonium chloride, N,N-cetylethylmorpholinium ethosulfate and trioctylmethylammonium chloride.

Especially preferred as dienes are myrcene, isoprene and ocimene and in this case the halogen is chlorine and the catalyst is a copper catalyst.

The process is especially carried out at a temperature from about −30°C to 50°C.

The preferred catalyst is cuprous chloride.

The catalytic proportion of copper catalyst should be within the weight range of 0.01 to 10.0 percent of the dry diene, and the hydrohalogenation is especially carried out for a period of from 3 to 15 hours.

## BRIEF DESCRIPTION OF THE DRAWING

The accompanying drawing is a schematic representation of a preferred embodiment process of the invention.

## DETAILED DESCRIPTION OF THE PREFERRED EMBODIMENTS OF THE INVENTION

The process of the invention may be employed for the hydrohalogenation of any conjugated diene. Representative of such dienes are myrcene, isoprene, 2-methylpiperylene, *beta*-phellandrene, 2-ethylbutadiene, ocimene, alloocimene, 1-phenylbutadiene, and the like.

The method of the invention is particularly advantageous for the hydrohalogenation of myrcene to obtain the geranyl and neryl halides which are intermediates for the manufacture of commercially valuable geraniol and nerol. When applied to myrcene, the improved method of the invention will increase the overall yield of the geranyl and neryl halides by at least 11 to 12 mole percent over the prior art processes and will improve selectivity of the ratio of geranyl isomer to the neryl isomer from about 1.3 to 1.6. Commercially available myrcene made by pyrolysis of *beta*-pinene, purified forms of myrcene, and myrcene isolated from natural materials may be provided as the starting material in the preferred process of the invention.

The accompanying drawing is a schematic representation of a preferred embodiment method of the invention for the hydrochlorination of myrcene. As shown in the Figure the provided myrcene initially held in tank 10 may be first dried in a conventional salt bed dryer 20 to remove water, as necessary. The dried myrcene is then preferably cooled to a temperature in the range of from about −30°C to about 30°C; most preferably circa 10°C in a cooling unit 30. Alternatively, the myrcene may be cooled first and then processed through a salt bed dryer 20 to remove water. Cooling the starting myrcene prior to drying in the salt bed dryer 20 is somewhat advantageous in that pre-cooling increases drying efficiency in the salt bed dryer 20.

As shown in the Figure the dried and cooled myrcene starting material may be passed into a hydrohalogenation apparatus which comprises in the preferred embodiment a stirred tank reactor 40. In reactor 40, hydrohalogenation of the introduced myrcene is carried out in the presence of a catalytic proportion of a hydrohalogenation catalyst, at a temperature within the range of from about −30°C to about 50°C; preferably at a temperature within the range of from −10°C to 25°C, most preferably about 10°C. Hydrohalogenation may be effected, for example, by reaction of the myrcene with a hydrogen halide like hydrogen chloride or hydrogen bromide, preferably in substantially anhydrous form and under substantially anhydrous conditions, i.e. having less than about 5% water present in the reaction mixture. As shown in the Figure, the preferred hydrohalogenation is with gaseous hydrogen chloride which is introduced as a gas possibly generated in a vaporizer, and then metered into the reactor 40, via appropriate conduits. Advantageously, the hydrogen chloride is metered into the reactor 40 at a rate of from about 2.0 to about 300 gms/hour/mole of myrcene present in the reactor 40. Preferably, the rate is from about 4.0 to 8.0 gms/hour/mole of myrcene.

The organic quaternary salt is introduced into the reactor 40 from storage vessel 50. Myrcene, quaternary salt, hydrogen chloride and copper catalyst are introduced into the reactor 40 sequentially. Preferably, the reactor 40 after purging with an inert gas such as nitrogen is first charged with the cool myrcene, the copper catalyst, and the organic quaternary salt. While cooling and stirring, the hydrogen chloride is added incrementally to the charge.

A wide variety of catalysts for hydrohalogenation of myrcene are well known and include, for example, any copper compound having a valency of 2 or less, including metallic copper. Any copper compound convertible to the halide such as the bromide, iodide or chloride under conditions of the reaction may also be used. Representative of copper catalyst advantageously employed are the chloride, bromide, carbonate, oxide, acetate, formate, sulfate, and like derivative cupric and cuprous compounds. Preferred as the hydrochlorination catalyst in the improved process of the invention is cuprous chloride. Catalytic proportions of the anhydrous hydrohalogenation catalyst are generally within the weight range of from about 0.01 to 10 percent of the dry myrcene, preferably about 0.5 percent.

Organic quaternary salts are generally well-known in the art as is their preparation and include quaternary salts and quaternary resins where the central atom is nitrogen, phosphorus or arsenic.

Representative of organic quaternary salts which may be used in the process of the invention are those of the formula:—

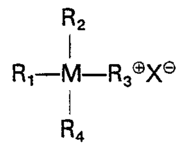

(VII)

wherein X is selected from the group consisting of an organic and inorganic anion such as nitrate, benzoate, phenylacetate, hydroxybenzoate, phenoxide, hydroxide, cyanide, nitrite; particularly preferred are chloride, bromide, iodide, methyl sulfate, ethyl sulfate and the like; M represents nitrogen, arsenic, or phosphorus. $R_1$, $R_2$, $R_3$ and $R_4$ are each independently selected from one of those groups consisting of hydrocarbyl and substituted hydrocarbyl or $R_1$ and $R_2$ may be taken together to represent a divalent moiety attached to the atom M, and which is selected from the group consisting of alkenylene and hydrocarbyl-substituted alkenylene having 5 to 10 carbon atoms, inclusive, in the ring thereof; or $R_1$ and $R_2$ may be taken together with the atom of M to which they are attached to represent a divalent or monovalent moiety selected from the groups consisting of those having the formula:—

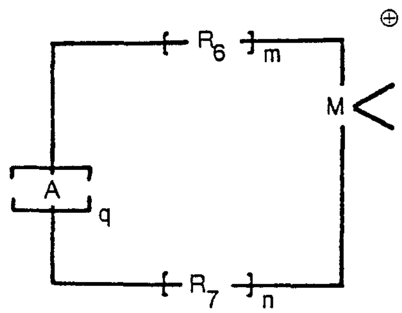

wherein A represents nitogen, oxygen, sulfur, phosphorus and the like; and $R_6$ and $R_7$ are each selected from alkenylene and hydrocarbyl-substituted alkenylene of 1 to 25 carbon atoms, inclusive; m, n and 1 are each integers of 0 to 1 and the sum of m+n is 1 or 2.

Preferred organic quaternary salts employed in the method of the invention will have a carbon atom content of at least 18 carbon atoms.

The term "hydrocarbyl" as used herein means the monovalent moiety obtained upon removal of a hydrogen atom from a parent hydrocarbon. Representative of hydrocarbyl are alkyl of 1 to 25 carbon atoms, inclusive, such as methyl, ethyl, propyl, butyl, pentyl, hexyl, heptyl, octyl, nonyl, indexyl, decyl, dodecyl, octadecyl, nonodecyl, eicosyl, heneicosyl, docosyl, tricosyl, tetracosyl, pentacosyl and the isomeric forms thereof; aryl of 6 to 25 carbon atoms, inclusive, such as phenyl, tolyl, xylyl, naphthyl, biphenyl, tetraphenyl and the like; aralkyl of 7 to 25 carbon atoms, inclusive, such as benzyl, phenethyl, phenpropyl, phenbutyl phenhexyl, napthoctyl and the like; cycloalkyl of 3 to 8 carbon atoms, inclusive, such as cyclopropyl, cyclobutyl, cyclopentyl, cyclohexyl, cycloheptyl, cyclooctyl and the like; alkenyl of 2 to 25 carbon atoms, inclusive, such as vinyl, allyl, butenyl, pentenyl, hexenyl, octenyl, nonenyl, decenyl, undececyl, dodecenyl, tridecenyl, pentadecenyl, octadecenyl, pentacosynyl and isomeric forms thereof.

The term "alkenylene" means the divalent moiety obtained on removal of two hydrogen atoms, each from a non-adjacent carbon atom of a parent hydrocarbon and includes alkenylene of 3 to 10 carbon atoms, inclusive, such as 1,3-propenylene, 1,4-butenylene, 1,5-pentenylene, 1,8-octylene, 1,10-decenylene and the like.

The terms "substituted hydrocarbyl" and "substituted alkenylene" as used herein mean the hydrocarbyl or alkenylene moiety as previously defined wherein one or more hydrogen atoms have been replaced with an inert group, i.e. a chemical group which does not adversely affect the desired function of the organic quaternary salt of formula (VII). Representative of such groups are aminophosphino-, hydrocarbyl, quaternary nitrogen (ammonium), quaternary phosphorus (phosphonium), hydroxyl-, alkoxy, mercapto-, alkyl, halo-, phosphate, phosphite, carboxylate groups and the like.

Organic quaternary compounds of the formula (VII) given above are generally well-known as are methods of their preparation. Representative of such organic quaternary compounds are trioctylmethylammonium chloride, tetraoctadecylammonium chloride, dodecyldimethylbenzylammonium chloride, tetradecyldimethylbenzylammonium chloride, hexadecyldimethylbenzylammonium chloride, trimethylcarboxymethylammonium chloride, triethylgeranylammonium chloride, triethylnerylammonium chloride, N.N-cetylethylmorpholinium ethosulfate, methyl(1)cocoamidoethyl(2)cocoimidazolinium methyl sulfate, N-tallow-pentamethylpropanediammonium dichloride, trioctylmethylphosphonium bromide, N,N-soya ethylmorpholinium ethosulfate, hexadecylpyridinium chloride, triethylbenzylammonium chloride, benzyl

hydroxyethyl(2)-cocoimidazolinium chloride, tripropylgeranylammonium chloride, tributylgeranylammonium chloride, dodecyldimethyl(ethylbenzyl)ammonium chloride, tetradecyldimethyl-(ethylbenzyl)ammonium chloride, hexadecyldimethyl(ethylbenzyl)ammonium chloride, octadecyldimethyl(ethylbenzyl)ammonium chloride, octadecyldimethylbenzylammonium chloride, methyl bis(2-hydroxyethyl)cocoammonium chloride, methyl(1)soyaamidoethyl(2)soyaimidazolinium methyl sulfate, methyl(1)tallowamidoethyl(2)tallow imidazolinium methyl sulfate, methyl(1)oleylamidoethyl(2)oleylimidazolinium methyl sulfate and the like.

Commercially available quaternary salts or purified forms of quaternary salts may be used in the preferred process of the invention.

It will be appreciated that under specific conditions of operating the process of the invention, certain of the above described compounds of the formula (VII) given above have advantages over other catalysts of the same general formula. Selection of a particular compound (VII) for use under specific process conditions, for optimum yields may be made by trial and error technique. We have observed however that there are advantages associated with a mixture of trialkylmethylammonium chlorides where the alkyl portion consists of a chain of from eight to ten carbon atoms. For example, Adogen 464 (Sherex Chemical Co.) and Aliquat 336 (Henkel Corp.).

The organic quaternary salt is used in a proportion to isomerize, during the hydrohalogenation reaction, at least some of the more-substituted allylic chloride produced in the method of the invention. Such a proportion is generally within the range of from about 0.01 to 10 percent by weight of the diene charge, preferably 0.2 to 2.5 percent. Optimum proportions will depend to some extent upon the salt selected and may be determined by trial and error technique.

The controlling reaction rate in the hydrohalogenation process of the invention is the isomerization of the more-substituted halide to the desired less-substituted halide. This is controlled by residence time in the hydrohalogenation reaction zone. We have found that in hydrochlorination of myrcene, the preferred minimum total residence time is within the range of from 3 to 15 hours, and most preferably 5 to 8 hours under the above described operating temperatures. The presence of linalyl chloride in the reaction mixture may be monitored by conventional analytical techniques. Longer residence times in the hydrochlorination reactor may cause a yield loss due to conversion of the monochlorides to *alpha*-terpinyl chloride. Shorter residence times may not be sufficient to isomerize the linalyl chloride to the desired geranyl/neryl chlorides.

When it has been determined that hydrohalogenation has occurred to a maximum desired point, the hydrohalogenation product mixture is passed from the hydrohalogenation apparatus. The desired hydrohalogenated diene may be separated from the reaction mixture employing conventional and known techniques including washing, decantation, distillation and like techniques.

The following examples describe the manner and process of making and using the invention and set forth the best mode contemplated by the invention and set forth the best mode contemplated by the inventors of carrying out the invention but are not to be construed as limiting. All parts given are by weight unless otherwise indicated.

## Example 1

To a 1 liter reaction vessel was charged 700.0 g myrcene (72 wt %), 2.2 g cuprous chloride and 10.5 g Adogen 464; supra. The mixture was purged with nitrogen and cooled to 0°C. Hydrogen chloride gas was added at a rate of 20 g/hour while maintaining the temperature at 10°C. At the end of the reaction (7 hours) monitored by infrared spectroscopy to a 1% myrcene level, the reaction product was neutralized with sodium carbonate and aqueous sodium hydroxide. The product was analyzed by gas chromatography.

The result of this analysis was as follows: molar yield from myrcene (%) — linalyl chloride 8, geranyl chloride 51, neryl chloride 32; geranyl to neryl chloride ratio — 1.59.

## Examples 2—7

The same starting material used in Example 1 was used and subjected to hydrochlorination under the same conditions as described in Example 1 using 2.2 g cuprous chloride and a quaternary salt as described in Table 1 to obtain the results shown in Table 1. Linalyl, neryl and geranyl chlorides are abbreviated as LCl, NCl and GCl respectively.

TABLE 1

| Example No. | Quaternary Salt and Amount Used | Molar Yield of Products (%) LCI | NCI+GCI | GCI:NCI Ratio |
|---|---|---|---|---|
| 2 | None | 18 | 69 | 1.26 |
| 3 | Benzyldimethylstearyl ammonium chloride, 9.4 g | 8 | 79 | 1.69 |
| 4 | Benzylhydroxyethyl(2)coco imidazolium chloride, 8.2 g | 7 | 73 | 1.40 |
| 5 | Methyltrioctylammonium chloride, 10.5 g | 9 | 74 | 1.52 |
| 6 | Tetra(octadecyl)ammonium bromide, 8.0 g | 16 | 73 | 1.18 |
| 7 | N,N-Cetylethylmorpholinium ethosulfate, 10.1 g | 8 | 70 | 1.39 |

Example 8

To a chilled 100 ml reaction vessel was charged 34.0 g isoprene, 0.3 g cuprous chloride and Adogen 464; supra. in an amount indicated in Table 2. The mixture was cooled to 0°C. Hydrogen chloride gas was added at a rate of 7 g/hour while maintaining the temperature at 0°C. At the end of reaction (3 hours), monmitored by weight, the reaction product was neutralized and analyzed by nuclear magnetic resonance spectroscopy to determine the amounts of 3-chloro-3-methyl-1-butene and 1-chloro-3-methyl-2-butene (abbreviated 3,3,1-B and 1,3,2-B respectively. The result of this analysis is shown in Table 2.

TABLE 2

| Amount Adogen 464 used | Composition of Products (%) 3,3,1-B | 1,3,2-B |
|---|---|---|
| None | 28 | 72 |
| 1.3 g | 11 | 89 |

Example 9

To a chilled 50 ml reaction vessel was charged 10.0 g 2-methylpiperylene, 0.07 g cuprous chloride and Adogen 464 (supra) in an amount indicated in Table 3. The mixture was cooled to 0°C. Hydrogen chloride gas was added at a rate of 1.5 g/hour while maintaining the temperature at 0°C. At the end of reaction (3 hours) monitored by weight, the reaction product was neutralized and analyzed by nuclear magnetic resonance spectroscopy and gas chromatography to determine the amounts of 4-chloro-4-methyl-2-pentene and 4-chloro-2-methyl-2-pentene (abbreviated 4,4,2-P and 4,2,2-P respectively). The result of this analysis is shown in Table 3.

TABLE 3

| Amount Adogen 464 used | Composition of Products (%) 4,4,2-P | 4,2,2-P |
|---|---|---|
| None | 26 | 74 |
| 0.3 g | 19 | 81 |

## Claims

1. In a method for the hydrohalogenation of a conjugated diene which comprises hydrohalogenating the diene in the presence of a catalytic proportion of a hydrohalogenation catalyst, the improvement which comprises; carrying out the hydrohalogenation in the presence of an organic quaternary salt.

2. The improved method of claim 1 where the organic quaternary salt has a carbon atom content of at least 18.

3. The improved method of claim 2 wherein the organic quaternary salt is troctylmethylammonium chloride.

4. The improved method of claim 1 wherein the organic quaternary salt is a mixture of tri(alkyl)methylammonium chlorides where the alkyl portion comprises of a chain of from eight to ten carbon atoms.

5. The improved method of claim 2 wherein the organic quaternary salt is benzyldimethylstearylammonium chloride.

6. The improved method of claim 2 wherein the organic quaternary salt is benzylhdyroxyethyl(2)coco imidazolium chloride.

7. The improved method of claim 2 wherein the organic quaternary salt is tetra(octadecyl)ammonium chloride.

8. The improved method of claim 2 wherein the organic quaternary salt is N,N-cetylethylmorpholinium ethosulfate.

9. The improved method of claim 2 or 4 wherein the organic quaternary salt is trioctylmethylammonium chloride.

10. The improved method of one of the claims 1—9 wherein the diene is myrcene, the halogen is chlorine and the catalyst is a copper catalyst.

11. The improved method of claim 10 wherein the organic quaternary salt is a mixture of tri(alkyl)methylammonium chlorides where the alkyl portion comprises a chain of from 8 to 10 carbon atoms.

12. The improved method of one of the claims 1—9 wherein the diene is isoprene, the halogen is chlorine and the catalyst is a copper catalyst.

13. The improved method of one of the claims 1—9 wherein the diene is ocimene, the halogen is chlorine and the catalyst is a copper catalyst.

14. The improved process of one of the claims 1—13 wherein the temperature of reaction is from about −30°C to 50°C.

15. The process of one of the claims 1—14 wherein the catalyst is cuprous chloride.

16. The process of one of the claims 1—15 wherein the catalytic proportion of copper catalyst is within the weight range of 0.01 to 10.0 percent of the dry diene.

17. The process of one of the claims 1—16 wherein the hydrohalogenation is carried out for a period of from 3 to 15 hours.

## Patentansprüche

1. In einem Verfahren zur Hydrohalogenierung eines konjugierten Diens, wobei das Dien in Gegenwart eines katalytischen Anteils eines Hydrohalogenierungskatalysators hydrohalogeniert wird, besteht die Verbesserung in der Durchführung der Hydrohalogenierung in Gegenwart eines organischen quaternären Salzes.

2. Verbessertes Verfahren nach Anspruch 1, wobei das organische quaternäre Salz mindestens 18 Kohlenstoffatome enthält.

3. Verbessertes Verfahren nach Anspruch 2, wobei das organische quaternäre Salz Trioctylmethylammoniumchlorid ist.

4. Verbessertes Verfahren nach Anspruch 1, wobei das organische quaternäre Salz ein Gemisch aus Tri(alkyl)methylammoniumchloriden ist, deren Alkylanteil aus einer Kette mit 8 bis 10 Kohlenstoffatomen besteht.

5. Verbessertes Verfahren nach Anspruch 2, wobei das organische quaternäre Salz Benzyldimethylstearylammoniumchlorid ist.

6. Verbessertes Verfahren nach Anspruch 2, wobei das organische quaternäre Salz Benzylhydroxyethyl(2)-coco-imidazoiliumchlorid ist.

7. Verbessertes Verfahren nach Anspruch 2, wobei das organische quaternäre Salz Tetra(octadecyl)ammoniumchlorid ist.

8. Verbessertes Verfahren nach Anspruch 2, wobei das organische quaternäre Salz N,N-Cetylethylmorpholiniumethosulfat ist.

9. Verbessertes Verfahren nach Anspruch 2 oder 4, wobei das organische quaternäre Salz Trioctylmethylammoniumchlorid ist.

10. Verbessertes Verfahren nach einem der Ansprüche 1—9, wobei das Dien Myrcen, das Halogen Chlor und der Katalysator ein Kupferkatalysator ist.

11. Verbessertes Verfahren nach Anspruch 10, wobei das organische quaternäre Salz ein Gemisch aus

Tri(alkyl)methylammoniumchloriden ist, deren Alkylanteil aus einer Kette mit 8 bis 10 Kohlenstoffatomen besteht.

12. Verbessertes Verfahren nach einem der Ansprüche 1—9, wobei das Dien Isopren, das Halogen Chlor und der Katalysator ein Kupferkatalysator ist.

13. Verbessertes Verfahren nach einem der Ansprüche 1—9, wobei das Dien Ocimen, das Halogen Chlor und der Katalysator ein Kupferkatalysator ist.

14. Verbessertes Verfahren nach einem der Ansprüche 1—13, wobei die Reaktionstemperatur zwischen etwa −30°C und 50°C beträgt.

15. Verfahren nach einem der Ansprüche 1—14, wobei der Katalysator Kupfer(I)-chlorid ist.

16. Verfahren nach einem der Ansprüche 1—15, wobei der katalytische Anteil des Kupferkatalysators in einem Gewichtsbereich zischen 0,01 und 10%, bezogen auf das trockene Dien, vorliegt.

17. Verfahren nach einem der Ansprüche 1—16, wobei die Hydrohalogenierung während eines Zeitraums von 3 bis 15 Stunden durchgeführt wird.

**Revendications**

1. Dans un procédé d'hydrohalogénation d'un diène conjugé qui consiste à hydrohalogéner le diène en présence d'une proportion catalytique d'un catalyseur d'hydrohalogénation, l'amélioration qui consiste à effecteur cette hydrohalogénation en présence d'un sel quaternaire organique.

2. Le procédé d'amélioration selon la revendication 1, dans lequel le sel quaternaire organique comporte au moins 18 atomes de carbone.

3. Le procédé d'amélioration selon la revendication 2, dans lequel le sel quaternaire organique est le chlorure de trioctylméthylammonium.

4. Le procédé d'amélioration selon la revendication 1, dans lequel le sel quaternaire organique est un mélange de chlorures de tri(alkyl)méthylammonium dans lequel la portiom alkyle est constituée d'une chaîne renfermant de 8 à 10 atomes de carbone.

5. Le procédé d'amélioration selon la revendication 2, dans lequel le sel quaternaire organique est le chlorure de benzyldiméthylstéarylammonium.

6. Le procédé d'amélioration selon la revendication 2, dans lequel le sel quaternaire organique est le chlorure de benzylhydroxyéthyl(2)coco-imidazolium.

7. Le procédé d'amélioration selon la revendication 2, dans lequel le sel quaternaire organique est le chlorure de tétra(octadécyl)ammonium.

8. Le procédé d'amélioration selon la revendication 2, dans lequel le sel quaternaire organique est l'éthosulfate de N,N-cétyléthylmorpholinium.

9. Le procédé d'amélioration selon la revendication 2 ou 4, dans lequel le sel quaternaire organique est le chlorure de trioctylméthylammonium.

10. Le procédé d'amélioration selon la quelconque des revendications 1 à 9, dans lequel le diène est le myrcène, h'halogène est le chlore et le catalyseur est un catalyseur à base de cuivre.

11. Le procédé d'amélioration selon la revendication 10, dans lequel le sel quaternaire organique est un mélange de chlorures de tri(alkyl)méthylammonium dans lequel la portion alkyle est formée d'une chaîne renfermant de 8 à 10 atomes de carbone.

12. Le procédé d'amélioration selon l'une quelconque des revendications 1 à 9, dans lequel le diène est l'isoprène, l'halogène est le chlore et le catalyseur est un catalyseur à base de cuivre.

13. Le procédé d'amélioration selon l'une quelconque des revendications 1 à 9, dans lequel le diène est l'ocimène, l'halogène est le chlore et le catalyseur est un catalyseur à base de cuivre.

14. Le procédé d'amélioration selon l'une quelconque des revendications 1 à 13, dans lequel la température de réaction est comprise entre environ −30°C et 50°C.

15. Le procédé selon l'une quelconque des revendications 1 à 14, dans lequel le catalyseur est le chlorure cuivreux.

16. Le procédé selon l'une quelconque des revendications 1 à 15, dans lequel la proportion catalytique de catalyseur à base de cuivre est comprise dans l'intervalle pondéral de 0,01 à 10,0% par rapport au poids du diène sec.

17. Le procédé selon l'une quelconque des revendications 1 à 16, dans lequel l'hydrohalogénation est mise en oeuvre pendant une durée de 3 à 15 heures.

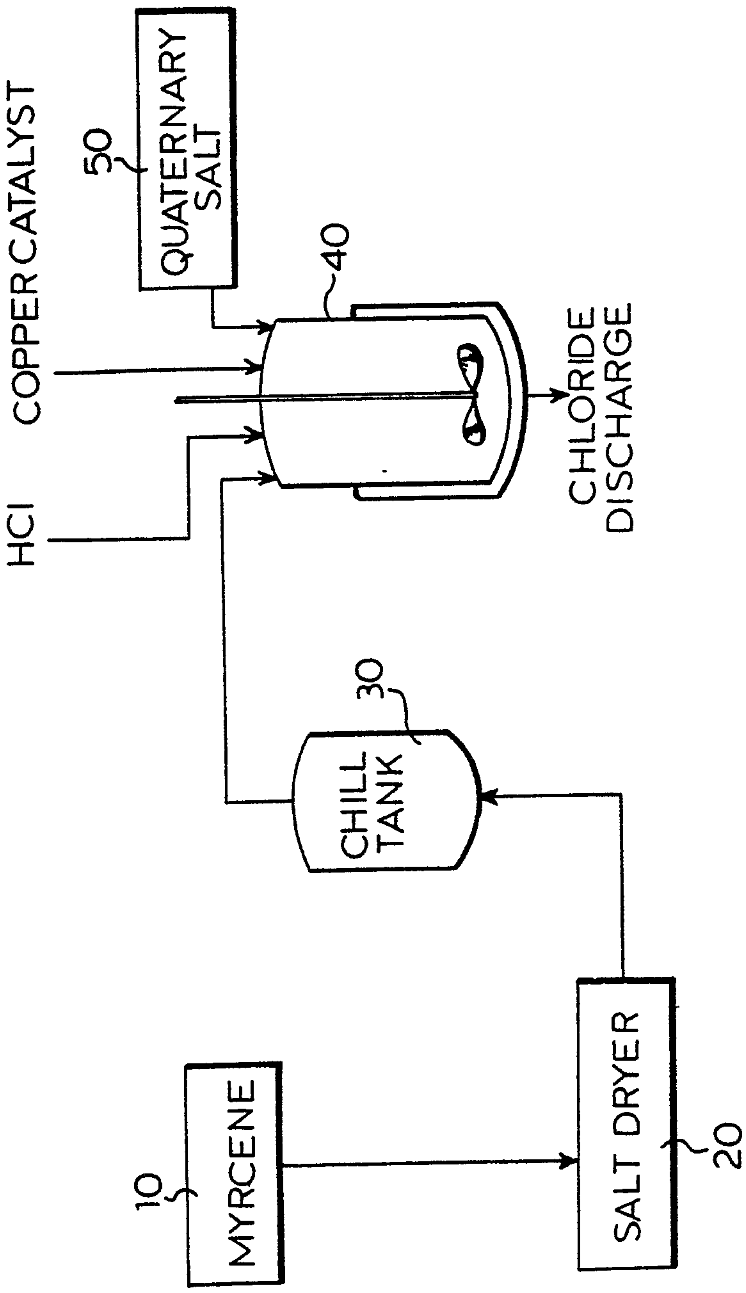
MYRCENE
10
SALT DRYER
20
CHILL TANK
30
HCl
COPPER CATALYST
QUATERNARY SALT
50
40
CHLORIDE DISCHARGE